# EUROPEAN PATENT SPECIFICATION

(11) **EP 3 856 206 B1**
(45) Date of publication and mention of the grant of the patent: **22.04.2026**
(21) Application number: 19786671.8
(22) Date of filing: 26.09.2019
(51) Int. Cl.: A61K 35/17, A61K 31/675, A61P 35/00

(54) **METHODS AND COMPOSITIONS FOR THE EXPANSION AND USE OF ALLOGENEIC GAMMA/DELTA-T CELLS**
VERFAHREN UND ZUSAMMENSETZUNGEN ZUR EXPANSION UND VERWENDUNG ALLOGENER GAMMA-/DELTA-T-ZELLEN
PROCÉDÉS ET COMPOSITIONS POUR LA MULTIPLICATION ET L'UTILISATION DE LYMPHOCYTES T GAMMA/DELTA ALLOGÉNIQUES

(30) Priority: 27.09.2018 US 201862737378 P
(43) Date of publication of application: 04.08.2021
(73) Proprietor: Phosphogam, Inc., Durham NC 27705 (US)
(72) Inventor: LOPEZ, Richard D., Durham, North Carolina 27705 (US)
(74) Representative: Inspicos P/S
(86) International application number: PCT/IB2019/058188
(87) International publication number: WO 2020/065584

(56) References cited:
- EP-A1- 1 878 440
- WO-A1-2014/072446
- MOHANADH NADA ET AL: "Enhancing adoptive cancer immunotherapy with V[gamma]2V[delta]2 T cells through pulse zoledronate stimulation", JOURNAL FOR IMMUNOTHERAPY OF CANCER, BIOMED CENTRAL LTD, LONDON, UK, vol. 5, no. 1, 21 February 2017 (2017-02-21), pages 1 - 23, XP021242440, DOI: 10.1186/S40425-017-0209-6
- MAKOTO KONDO ET AL: "Expansion of Human Peripheral Blood [gamma][delta] T Cells using Zoledronate", JOURNAL OF VISUALIZED EXPERIMENTS, no. 55, 9 September 2011 (2011-09-09), XP055650691, DOI: 10.3791/3182
- ZHENG XIANG ET AL: "Dual Face of V[gamma]9V[delta]2-T Cells in Tumor Immunology: Anti- versus Pro-Tumoral Activities", FRONTIERS IN IMMUNOLOGY, vol. 8, 28 August 2017 (2017-08-28), CH, XP055650681, ISSN: 1664-3224, DOI: 10.3389/fimmu.2017.01041

## Description

### FIELD OF THE INVENTION

This invention relates to the field of immunology and methods of treating cancer.

### BACKGROUND OF THE INVENTION

Unlike classical (conventional) αβ-T cells that recognize specific peptide antigens presented by major histocompatibility complex (MHC) molecules, γδ-T cells (gamma/delta-T cells) in contrast appear to recognize generic determinants expressed by cells that have become dysregulated as a result of either malignant transformation or viral infection (Kabelitz D. et al. Cancer Res. 2007;67(1):5-8; Silva-Santos B, et al., Eur J Immunol. 2012;42(12):3101-5; Vantourout P and Hayday A. Nature reviews Immunology 2013;13(2):88-100). γδ-T cells have the innate ability to recognize and kill a broad spectrum of tumor cell types, in a manner that does not require the existence of bona fide tumor-specific antigens. As such, there is a need to develop therapeutic strategies to target γδ-T cells against a variety of cancers. Nada, M. H., et al. (J. Immunother. Cancer. 2017;5:9) discloses treatment of cancer in mice by adoptive transfer of human γδ-T cells into the mice, wherein the mice were pre-treated with the amino-bisphosphonate pamidronate before the adoptive transfer, and wherein the adoptively transferred cells had been expanded *ex vivo* using zoledronate.

### SUMMARY OF THE INVENTION

The invention is defined by the appended claims.

In a first aspect, the invention relates to donor-derived allogeneic γδ-T cells for use in a method for treating cancer in a human subject, the method comprising:
(a) administering at least one lymphodepletion treatment or a Hematopoietic Stem Cell (HSC) transplant to the subject;
(b) administering zoledronic acid to the subject; and,
(c) subsequently administering at least one dose of the donor-derived

allogeneic γδ-T cells to the subject, wherein the γδ-T cells are expanded ex vivo by culturing the γδ-T cells with at least one γδ-T cell expansion compound prior to administration of the γδ-T cells to the subject;
wherein the at least one γδ-T cell expansion compound comprises C-HDMAPP;
wherein the treatment results in an antitumor response in the subject.

In some embodiments, the lymphodepletion treatment comprises administering one or more chemotherapeutic agents or low dose total body irradiation. In further embodiments, the one or more chemotherapeutic agents comprise cyclophosphamide, fludarabine or melphalan.

In some embodiments, the method further comprises administering to the subject: a) one or more of etoposide, cisplatin, doxorubicin, 5-Fluorouracil, vincristine, bortezomib, oxaliplatin or ibrutinib; b) a therapeutically effective amount of at least one monoclonal antibody therapy; and/or c) one or more additional cellular therapies. In further embodiments, the monoclonal antibody recognizes a tumor antigen. In other embodiments, the one or more additional cellular therapies are from the same allogeneic donor as the γδ-T cells.

In some embodiments, the method further comprises culturing the γδ-T cells with interleukin-2.

In some embodiments, the method further comprises culturing the γδ-T cells with one or more checkpoint inhibitors prior to administration of the γδ-T cells to the subject. In further embodiments, the one or more checkpoint inhibitors comprises an anti-PD-1 antibody, an anti-PDL1 antibody, an anti-TIM-3 antibody, an anti-LAG3 antibody, an anti-galaectin-9 antibody, an anti-IDO antibody and/or an anti-VISTA antibody.

In some embodiments, the method further comprises culturing the γδ-T cells with one or more of IL-15, an anti-CD277 antibody, an anti-TGF-beta antibody, a prostaglandin E2 inhibitor, adenosine, osteopontin, vitamin C and/or a hypomethylating agent prior to administration of the γδ-T cells to the subject.

In some embodiments, the subject and the donor of the γδ-T cells have a full human leukocyte antigen (HLA) mismatch.

In some embodiments, the method further comprises repeating steps (a) and (c) one or more times, wherein the donor-derived allogeneic γδ-T cells for each subsequent administration are from a different donor having a full HLA mismatch as compared to the subject and the previous donor(s).

References to methods of treatment by therapy with cells and/or other agents are to be interpreted as references to the cells and/or other agents for use in those methods.

### BRIEF DESCRIPTION OF THE FIGURES

FIG. 1 provides flow cytometry analysis of the ex vivo expanded donor-derived allogeneic γδ-T cells cultured for 21 days in various nM concentrations of the synthetic phosphoantigen C-HDMPP.
FIG. 2 shows the fold expansion over time of the donor-derived allogeneic γδ-T cells cultured with 100 nM C-HDMPP.
FIG. 3 demonstrates the in vitro anti-tumor activity of the ex vivo expanded human γδ-T cells against human tumor cell lines. Data are shown as tumor cell lysis at the E:T ratio of 25:1.
FIG. 4 depicts that pre-treatment of tumor cells for 24 hours with zoledronic acid (ZOL) can render relatively resistant MDA-MB-231 tumor cells more sensitive to killing by the ex vivo expanded γδ-T cells. Data are shown as tumor cells lysis at the indicated E:T ratios.
FIG. 5 demonstrates that pre-treatment of various different tumor cells for 24 hours with zoledronic acid (ZOL) can render relatively resistant tumor cells more sensitive to killing by the ex vivo expanded γδ-T cells. Data are shown as tumor cell lysis at the indicated E:T ratios.

### DETAILED DESCRIPTION OF THE INVENTION

### I. Overview

Herein provided are methods of treating a cancer using various immunological maneuvers (treatments) to make subjects receptive to the subsequent clinical infusion of allogeneic γδ-T cells (gamma/delta-T cells) obtained from the blood of otherwise healthy donors. The introduction of allogeneic γδ-T cells obtained from healthy donors is intended to replace or augment the tumor-killing or infection-killing γδ-T cells that have otherwise been rendered non-functional within subjects.

As used herein, "γδ-T cells" or "gamma/delta-T cells" are any T cells that express a T cell receptor made up of one gamma chain and one delta chain.

### II. Methods of Treating Cancer

Provided herein are methods of treating cancer in a human subject. According to the first aspect of the invention, such methods comprise first administering a lymphodepletion treatment or a Hematopoietic Stem Cell (HSC) transplant to a subject followed by administration of at least one dose of donor-derived allogeneic γδ-T cells to the subject.

By "treating" a subject with cancer is intended administration of a therapeutically effective amount of at least one dose of donor-derived allogeneic γδ-T cells to a subject that has cancer, where the purpose is to cure, heal, alleviate, relieve, alter, remedy, ameliorate, improve, or affect the condition or one or more symptoms of cancer.

As used herein, "subject" is intended any animal (i.e. mammals) such as, humans, primates, rodents, agricultural and domesticated animals such as, but not limited to, dogs, cats, cattle, horses, pigs, sheep, and the like, in which one desires to treat cancer. In the claimed invention, the subject is a human.

A therapeutically effective amount of at least one dose of donor-derived allogeneic γδ-T cells is provided for use in the methods provided herein. A "therapeutically effective amount," "therapeutically effective dose," or "effective amount" as used herein refers to that amount which provides a therapeutic effect for a given condition and administration regimen. Thus, the phrase "therapeutically effective amount" is used herein to mean an amount sufficient to cause an improvement in a clinically significant condition in the host. In particular aspects, a "therapeutically effective amount" refers to an amount of donor-derived allogeneic γδ-T cells that, when administered, brings about a positive therapeutic response with respect to treatment of a subject for a cancer. A positive therapeutic response in regard to treating a cancer includes curing or ameliorating the symptoms of the disease. In the present context, a deficit in the response of the subject can be evidenced by continuing or spreading of the cancer. An improvement in a clinically significant condition in the subject includes a decrease in the size of a tumor, increased necrosis of a tumor, clearance of the tumor from the host tissue, reduction or amelioration of metastasis, or a reduction in any symptom associated with the cancer.

An "antitumor response" refers to a positive therapeutic response in regard to treating a cancer and includes curing or ameliorating the symptoms of the disease. An antitumor response in the subject includes a decrease in the size of a tumor, increased necrosis of a tumor, clearance of the tumor from the host tissue, the presence of anti-tumor immune cells in the subject, the presence of immune cells in or adjacent to the tumor, reduction or amelioration of metastasis, or a reduction in any symptom associated with the cancer.

As demonstrated in the present invention, the ex vivo expanded allogeneic γδ-T cells display a broad antitumor reactivity and therefore can target a broad range of tumors. Non-limiting examples of types of cancer encompassed by the methods herein include, carcinomas, sarcomas, leukemia, lymphoma, B-cell cancers, breast cancer, colon cancer, lung cancer, bladder cancer, pancreatic cancer, ovarian cancer, prostate cancer, brain tumors, acute lymphoblastic leukemia, and bone cancer. In one embodiment, the cancer comprises breast cancer. In another embodiment, the cancer is a blood cancer. In a specific embodiment, the cancer is a B-cell cancer. The B-cell cancer can comprise, for example, non-Hodgkin lymphoma, Hodgkin lymphoma, chronic lymphocytic leukemia, diffuse large B-cell lymphoma, multiple myeloma, follicular lymphoma, mantle-cell lymphoma, Burkitt's lymphoma, acute lymphoblastic leukemia, marginal-zone lymphoma or lymphoplasmacytic lymphoma.

### A. Donor-Derived Allogeneic γδ-T cells and Expansion Thereof

The methods provided herein comprise administering donor-derived allogeneic γδ-T cells. When compared to γδ-T cells found in healthy donors, γδ-T cells found in tumor-bearing hosts appear to be substantially diminished in number, and/or are functionally impaired in a variety of important ways. Thus, in the majority of tumor-bearing hosts, the γδ-T cell compartment may be irreversibly damaged or exhausted, this occurring in a tumor-dependent manner. While the mechanisms accounting for these numeric or functional defects remain unknown, the potential consequences are clear: any strategy which relies upon harnessing the innate antitumor properties of autologous (i.e., subject-derived) γδ-T cells will ultimately prove to be ineffective given these defects found to exist within the γδ-T cell compartment of tumor-bearing hosts.

Herein provided is the novel method of using adoptively transferred allogeneic γδ-T cells for the immunotherapy of malignant diseases: cells that are obtained from otherwise healthy donors. As used herein "allogeneic γδ-T cells" refers to γδ-T cells obtained from an individual different from the subject for which they are intended for infusion. Indeed, key to this concept is that in contrast to γδ-T cells present in tumor-bearing hosts, γδ-T cells obtained from healthy donors are essentially undamaged and will be more effective against cancer cells. Moreover, as donor-derived tumor-reactive γδ-T cells can readily be expanded ex vivo from peripheral blood obtained from virtually all healthy donors, in theory a limitless supply of highly effective tumor-reactive γδ-T cells can be made available for subsequent (and repeated) adoptive transfer into tumor-bearing hosts. As used herein "donor-derived" is meant γδ-T cells obtained from an individual different from the subject to which they are to be administered.

In some embodiments, the donor of the allogeneic γδ-T cells is a full human leukocyte antigen (HLA) mismatch as compared to the subject receiving the allogeneic γδ-T cells. By full HLA mismatch is meant that the subject and the donor do not share any HLA antigens. In other embodiments, the donor is a partial HLA mismatch. By partial HLA mismatch is meant that at least one HLA antigen is different between the subject and the donor. In such methods, the subject will be screened for any pre-formed anti-HLA antibodies. If the subject is found to have pre-formed anti-HLA antibodies against the non-shared HLA of the donor, then the donor cells will not be used for that subject. In a preferred embodiment, the subject and the donor of the γδ-T cells have a full HLA mismatch.

Given the scarcity of γδ-T cells in the blood of any person (donor or subject), the aforementioned adoptive transfer of donor-derived allogeneic γδ-T cells becomes feasible only following the ex vivo expansion of sufficiently large numbers of γδ-T cells first obtained from healthy donors. These γδ-T cells, once obtained from a donor, are to be expanded using a variety of different methods including, but not limited to, methods we and others have described previously. See, for example, Lopez RD, et al. Blood. 2000;96(12):3827-37; Bennouna J, et al. Cancer Immunol Immunother. 2008; 57(11): 1599-609; Bompas E, et al. ASCO Meeting Abstracts. 2006;24(18):2550; Espinosa E, et al. Journal of Biological Chemistry. 2001;276(21):18337-44; Gertner-Dardenne J, et al. Blood. 2009; Rojas RE, et al. Infection & Immunity. 2002;70(8):4019-27; Sicard H, et al. J Immunol. 2005;175(8):5471-80; and Squiban PJ, et al. J Clin Oncol 2007; 25(18suppl):3064.

Key to this concept is that in contrast to γδ-T cells present in tumor-bearing hosts (i.e., patients), γδ-T cells obtained from healthy (allogeneic) donors do in fact expand, whereas γδ-T cells taken from tumor-bearing patients have lost their ability to expand and are far less effective at killing tumor cells even if they were to expand. Moreover, as donor-derived tumor-reactive γδ-T cells can readily be expanded ex vivo from peripheral blood obtained from virtually all healthy donors, in theory a limitless supply of highly effective tumor-reactive γδ-T cells can be made available for subsequent (and repeated) adoptive transfer into tumor-bearing hosts. Thus, in the methods provided herein, the donor-derived allogeneic γδ-T cells can be administered to the subject at least once, at least twice, at least three times, at least 5 times, at least 10 times, at least 20 times or as many times as necessary to induce an appropriate antitumor response.

In the methods provided herein according to the first aspect of the invention, the allogeneic donor-derived γδ-T cells are expanded ex vivo by culturing the γδ-T cells with at least one γδ-T cell expansion compound prior to administration of the γδ-T cells to a subject, wherein the at least one γδ-T cell expansion compound comprises C-HDMAPP. By "γδ-T cell expansion compound" is meant any compound that induces or enhances the expansion of γδ-T cells. In some cases, the γδ-T cells may also be cultured with additional activation compounds, such as, for example, interleukin-2 (IL-2).

Various methods have been described for the ex vivo expansion of human γδ-T cells. This includes, but is not limited to, older methods originally reported by R. Lopez described in WO1999046365 A1, as well newer methods such as that of Nieda more recently described in US20120107292 A1. Example 1 provides an exemplary method of ex vivo γδ-T cell expansion.

Various γδ-T cell expansion compounds have been developed. This includes the synthetic phosphoantigen (BrHPP; Phosphostim, US Patent No. 7,109,183; US Patent No. 7,625,879; and US Patent No. 6,660,723) specifically developed for either the *in vivo* activation of endogenous γδ-T cells when the compound is administered as a drug to a patient, or as means (method) to ex vivo expand γδ-T cells first collected from patients as described by Salot (US Publication No. 2005196385A1). Non-limiting examples of other yδ-T cell expansion compounds with the same intended clinical use include: the synthetic phosphoantigen C-HDMAPP ("Picostim") described in US Patent No. 7,399,756 and EP1408984; polymorphic forms of C-HDMAPP (WO2010029062); and "Mayoly" (US8017596B2). In addition, amino-bisphosphonates, such as pamidronate (Aredia) or zoledronate (Zometa), also known as zoledronic acid, can also be used to expand γδ-T cells in the methods provided herein.

Amino-bisphosphonates act indirectly on γδ-T cells by causing bystander cells present in PBMC cultures to release isopentenyl pyrophosphate (IPP) into the culture which then induces the expansion of γδ-T cells. This can result in highly variable, inefficient and unpredictable γδ-T cell expansion. In contrast, the synthetic phosphoantigens, for example, C-HDMAPP, act directly on γδ-T cells, thereby allowing for precise concentration optimization and predictable expansion of γδ-T cells. In the claimed invention, the at least one γδ-T cell expansion compound comprises C-HDMAPP.

In some embodiments, the γδ-T cell expansion compounds can be used in combination with other compounds. Such other compounds may enhance the expansion of γδ-T cells when used in combination with one or more γδ-T cell expansion compounds. Other compounds may enhance the survival of γδ-T cells during their ex vivo expansion. Still other compounds may enhance the in vivo survival of the γδ-T cells following their introduction into humans. Still other compounds may promote the preferential outgrowth of distinct functional phenotypes of γδ-T cells which are more potent in their ability to kill tumors, or are more able to home into sites of tumor within tissues given the preferential upregulation of specific homing receptors.

In one embodiment of the first aspect of the invention, the donor derived allogeneic γδ-T cells are expanded ex vivo by culturing the γδ-T cells with the at least one phosphoantigen and an anti-CD277 antibody. The anti-CD277 antibody can be any anti-CD277 antibody including, but not limited to, the anti-CD277 antibodies described in U.S. Application Publication No. 2015/0353643, such as the 20.1 antibody.

In other embodiments of the first aspect of the invention, the donor-derived allogeneic γδ-T cells are expanded ex vivo by culturing the γδ-T cells with the at least one phosphoantigen and one or more of the following: cytokines including, but not limited to, interleukin-2 (IL-2), IL-12, IL-15, IL-17, IL-33 and synthetic agonists thereof; immune checkpoint inhibitors, including, for example, checkpoint inhibitor antibodies including, but not limited to, the anti-CD277 20.1 antibody described in U.S. Application Publication No. 2015/0353643, anti-PD-1 antibodies, anti-PDL1 antibodies, anti-CTLA4 antibodies, antibodies against LAG3, antibodies against TIM-3, anti-galaectin-9 antibodies, anti-IDO antibodies and anti-VISTA antibodies; "epigenetic modifiers" including, but not limited to, decitabine (hypomethylating agent) and azacytadine (hypomethylating agent) and vitamin C; mTOR inhibitors including, but not limited to, rapamycin (aka sirolimus) and everolimus; and other miscellaneous additives including, but not limited to, antibodies to TGF-beta; Prostaglandin E₂ inhibitors, GM-CSF, G-CSF, and osteopontin.

In one embodiment, the donor-derived allogeneic γδ-T cells are cultured with interleukin-2 (IL-2). In another embodiment, the donor-derived allogeneic γδ-T cells are cultured with one or more checkpoint inhibitors prior to administration of the γδ-T cells to the subject. In a specific embodiment, the one or more checkpoint inhibitors comprises an anti-PD-1 antibody, an anti-PDL1 antibody, an anti-TIM-3 antibody, an anti-LAG3 antibody, and anti-galaectin-9 antibody, an anti-IDO antibody and/or an anti-VISTA antibody.

In another embodiment, the donor-derived allogeneic γδ-T cells are cultured with one or more of IL-15, an anti-CD277 antibody, an anti-TGF-beta antibody, a prostaglandin E₂ inhibitor, adenosine, osteopontin, vitamin C and/or a hypomethylating agent prior to administration of the γδ-T cells to the subject.

None of the above mentioned compounds or methods were developed for use in the ex vivo expansion of allogeneic γδ-T cells obtained from healthy donors with the intent to subsequently transfer these cells into subjects as is provided in the therapeutic methods provided herein.

The tumor microenvironment (TME) is capable of suppressing the function of tumor-infiltrating lymphocytes, including γδ-T cells, which can lead to a loss or diminishment of anti-tumor immune responses. For cell-based immunotherapies in particular, a major challenge remains overcoming this TME-mediated immune suppression. The γδ-T cells may be modified post-expansion to directly or indirectly disrupt or inhibit specific immunosuppressive pathways within the TME. A variety of methods such as, but not limited to, gene editing, for example using CRISPR technologies, to make the γδ-T cells resistant to TME-mediated immunosuppression; disrupting receptors on γδ-T cells which respond to soluble immunosuppressive factors within the TME; or by disrupting receptors/ligands on γδ-T cells which bind to contact-dependent factors present on various cells within the TME. Non-limiting examples include disruption of receptors for TGF-beta or PD-1.

### B. Adoptive Transfer of Expanded Allogeneic Donor-Derived γδ-T cells

The methods of treating cancer provided herein require the subject to be made receptive to the allogeneic donor-derived γδ-T cells by performing an initial immunological maneuver or treatment. The means by which subjects are made receptive to donor-derived allogeneic γδ-T cells includes, but is not limited to, the performance of an allogeneic hematopoietic stem cell (HSC) transplant using HSC first obtained from a suitable bone marrow donor or by lymphodepletion with a lymphodepletion treatment. Without these initial immunological maneuvers (treatments), γδ-T cells of donor origin would be immediately rejected by a subject.

Provided herein are two distinct, yet related immunological strategies/platforms to specifically permit the adoptive transfer of tumor-reactive γδ-T cells from a healthy donor into a tumor-bearing host (subject).

### 1. Hematopoietic Stem Cell (HSC) Transplant Platform

The first platform for permitting the subsequent treatment of cancer through the adoptive transfer of donor-derived allogeneic γδ-T cells for use in the methods provided herein comprises administering a Hematopoietic Stem Cell (HSC) transplant to a subject prior to administration of the donor-derived allogeneic γδ-T cells. As used herein, "Hematopoietic Stem Cell (HSC) transplant" refers to the transplantation of multipotent hematopoietic stem cells. The hematopoietic stem cells can be derived, for example, from bone marrow, peripheral blood, or umbilical cord blood.

In one embodiment of the first aspect of the invention, the method of treating cancer comprises first administering a Hematopoietic Stem Cell (HSC) transplant to the subject. In such a method, the subject is subsequently administered the at least one dose of donor-derived allogeneic γδ-T cells. The γδ-T cells used in such a method are expanded ex vivo by culturing with the at least one γδ-T cell expansion compound prior to administration of the γδ-T cells to the subject. As such, the treatment induces an antitumor response in the subject.

In another embodiment of the first aspect of the invention, the method of treating cancer comprises first administering a Hematopoietic Stem Cell (HSC) transplant to the subject. In such a method, the subject is subsequently administered a combination therapy of the at least one dose of donor-derived allogeneic γδ-T cells and at least one dose of a therapeutically effective amount of at least one monoclonal antibody therapy. The γδ-T cells used in such a method are expanded ex vivo by culturing with the at least one γδ-T cell expansion compound prior to administration of the γδ-T cells to the subject.

In another embodiment of the first aspect of the invention, the method of treating cancer comprises first administering a Hematopoietic Stem Cell (HSC) transplant to the subject. In such a method, the subject is subsequently administered the at least one dose of donor-derived allogeneic γδ-T cells. The γδ-T cells used in such a method are expanded ex vivo by culturing with the at least one phosphoantigen and an anti-CD277 antibody prior to administration of the γδ-T cells to the subject.

In conventional HSC transplantation, antitumor effects are provided by high-dose chemotherapy and/or radiation delivered as part of the transplant conditioning. However, it is also evident that secondary nonspecific immune-mediated graft-versus-tumor effects also contribute to disease control. Though the mechanisms by which this occurs are not well understood, it is evident that competent donor-derived (allogeneic) immune effector cells play a key role in the graft-versus-tumor effects seen in the setting of allogeneic HSC transplantation in selected diseases.

Indeed, given the powerful antitumor effects of donor-derived immunity, in certain diseases it is not uncommon following allogeneic HSC transplantation to deliver a donor lymphocyte infusion (DLI) in order to either induce (promote) or sustain remission after transplantation. This however is commonly performed by introducing crude, unfractionated preparations of donor-derived peripheral blood lymphocytes containing primarily αβ-T cells. Accordingly, and not unexpectedly, such maneuvers commonly result in the development of sometimes life-threatening graft-versus-host disease (GVHD) in the recipient (15).

Given the potent innate antitumor properties of γδ-T cells as well as their inability to cause GVHD, donor-derived allogeneic γδ-T cells administered as a highly purified γδ-T cell donor lymphocyte infusion (γδ-T cell DLI) following allogeneic HSC transplant is the ideal cell to be introduced in such a setting.

In essence then, the allogeneic HSC transplant procedure itself will be relegated to a supporting role serving as the therapeutic platform for the subsequent adoptive transfer of tumor-reactive, donor-derived allogeneic γδ-T cells (γδ-T cell DLI).

### 2. Lymphodepletion Platform

The second platform for permitting the subsequent treatment of cancer through the adoptive transfer of donor-derived allogeneic γδ-T cells for use in the methods provided herein comprises administering a lymphodepletion treatment to a subject prior to administration of donor-derived allogeneic γδ-T cells.

It is intended that through the intentional immunosuppression of subjects using a variety of treatments, including, but not limited to, the administration of chemotherapy or other chemical compounds as well as exposure to radiation, subjects can be made transiently receptive to donor-derived allogeneic γδ-T cells that retain potent antitumor properties.

As used herein, "lymphodepletion" is meant the depletion of lymphocytes in a subject. Lymphodepletion can be a depletion of at least 10%, at least 20%, at least 30%, at least 40%, at least 50%, at least 60%, at least 70%, at least 80%, at least 85%, at least 90%, at least 95%, at least 98%, at least 99% or more of the lymphocytes in a subject. By "lymphocytes" is meant a type of white blood cell that is part of the immune system. Lymphocytes can include, for example, various types of B-cells, T-cells or natural killer (NK) cells. Lymphodepletion can be accomplished by administration of at least one lymphodepletion treatment. A "lymphodepletion treatment" as used herein, is any treatment that results in lymphodepletion in a subject. Lymphodepletion treatments include, but are not limited to, for example, methylprednisolone, radiation, low dose total body irradiation, etoposide, cisplatin, doxorubicin, 5-Fluorouracil, vincristine, bortezomib, oxaliplatin, or any lymphodepleting chemotherapeutic agents, including cyclophosphamide, fludarabine or melphalan. In one embodiment, the lymphodepletion treatment comprises administering one or more chemotherapeutic agents. In specific embodiments, the chemotherapeutic agents comprise cyclophosphamide, fludarabine or melphalan. In another embodiment, the lymphodepletion treatment comprises low dose total body irradiation.

While the HSC transplant platform described above is clearly logical, from a clinical perspective, such an approach which requires the performance of an allogeneic HSC transplant might not be appropriate for many subjects. Thus, a second platform, lymphodepletion, with potentially far greater clinical applicability, was developed.

The lymphodepletion platform establishes transient rather than permanent donor-host immunological tolerance prior to delivery of allogeneic γδ-T cells, thus making an allogeneic HSC transplant unnecessary.

The lymphodepletion platform is designed to accommodate the eventual rejection of adoptively transferred donor-derived allogeneic γδ-T cells. In the (transiently) lymphodepleted host, adoptively-transferred allogeneic γδ-T cells will still be able to mediate antitumor effects, particularly when delivered in a periodic and repeated fashion.

In pilot studies in animal models, we have determined that a functional window of opportunity can be established in which adoptively transferred allogeneic γδ-T cells can mediate measurable antitumor effects in lymphodepleted tumor-bearing hosts. In a clinically relevant manner, this functional window of opportunity can be created using, for example, cyclophosphamide, a drug that is both immunosuppressive (i.e., lymphodepleting) and under certain circumstances, effective against a variety of cancers.

In one embodiment of the first aspect of the invention, the method of treating cancer comprises first administering a lymphodepletion treatment to the subject. In such a method, the subject is subsequently administered the at least one dose of donor-derived allogeneic γδ-T cells. The γδ-T cells used in such a method are expanded ex vivo by culturing with the at least one γδ-T cell expansion compound prior to administration of the γδ-T cells to the subject.

In another embodiment of the first aspect of the invention, the method of treating cancer comprises first administering a lymphodepletion treatment to the subject. In such a method, the subject is subsequently administered a combination therapy of the at least one dose of donor-derived allogeneic γδ-T cells and at least one dose of a therapeutically effective amount of at least one monoclonal antibody therapy. The γδ-T cells used in such a method are expanded ex vivo by culturing with the at least one γδ-T cell expansion compound prior to administration of the γδ-T cells to the subject. As such, the combination therapy induces an antitumor response in the subject.

In another embodiment of the first aspect of the invention, the method of treating cancer comprises first administering a lymphodepletion treatment to the subject. In such a method, the subject is subsequently administered the at least one dose of donor-derived allogeneic γδ-T cells. The γδ-T cells used in such a method are expanded ex vivo by culturing with the at least one phosphoantigen and an anti-CD277 antibody prior to administration of the γδ-T cells to the subject.

In all the embodiments described above, a specific strategy for selecting donor cell products which are suitable for use in a given patient can involve screening donors and patients for human leukocyte antigen (HLA) type (both class I and II), wherein the cell products generated from each donor (which can be produced in bulk and then cryopreserved in single dose units) are well-characterized with respect to HLA; and/or screening patients for anti-HLA antibodies, using, for example, a standard Luminex-based assay. One strategy for selecting a compatible donor-derived γδ-T cell product for use in a given patient includes identifying a cell product derived from a donor as "suitable" or "compatible" for use in a given patient if the donor-derived cell product shares no HLA with the patient and/or if the patient has no anti-HLA antibodies directed against non-shared HLA of the donor (cell product) in order to avoid immediate antibody-mediated (humoral) rejection of donor cells.

The lymphodepletion treatment will allow γδ-T cells from an HLA-mismatched donor to be temporarily accepted. This will occur by the suppression of the host's cellular immune response which would otherwise recognize and immediately reject the HLA-mismatched γδ-T cells. Because the subject is screened for the absence of anti-HLA antibodies directed against the selected donor, no humoral (antibody-mediated) rejection of donor γδ-T cells will occur. As such, the lymphodepleted state will only be transient, and the subject will eventually recover immune function and reject a given HLA-mismatched donor's γδ-T cells. However, the present methods only require that HLA-mismatched γδ-T cells be present for a sufficiently long period of time in which they can kill tumor cells, before rejection.

Following rejection of the donor-derived allogeneic γδ-T cells, the lymphodepletion treatment followed by administration of allogeneic donor derived γδ-T cells may be repeated. In such methods, the lymphodepletion treatment will be administered to the subject again, but the administered γδ-T cells will be from a different HLA-mismatched donor. In some embodiments, the subject and the second γδ-T cell donor will have a full or partial HLA-mismatch. The cycle of lymphodepletion and administration of donor-derived allogeneic γδ-T cells may be repeated 1, 2, 3, 4, 5, 6, 7, 8, 9, 10, 11, 12, 13, 14, 15, 16, 17, 18, 19, 20 or more times. In such methods, each subsequent donor will also not share HLA with any of the previous donors.

In one embodiment of the first aspect of the invention, the method comprises administering at least one lymphodepletion treatment to the subject; and, subsequently administering the at least one dose of donor-derived allogeneic γδ-T cells to the subject, wherein the γδ-T cells are expanded ex vivo by culturing the γδ-T cells with the at least one γδ-T cell expansion compound prior to administration of the γδ-T cells to the subject, wherein the method is repeated one or more times and wherein the donor-derived γδ-T cells for each subsequent administration are from a different donor having a full HLA mismatch as compared to the subject and the previous donor(s).

A bank of γδ-T cells that maximizes the odds of a patient having a compatible cell product available can be generated by creating a repository of γδ-T cells derived from individuals who have uncommon HLA types, which increases the odds that any given patient does not share HLA with any given donor. The bank of γδ-T cells can also comprise γδ-T cells that have been pre-screened for their in vitro anti-tumor activity against a panel of human tumor cell lines. While the γδ-T cells will display killing against most tumor cell lines, cells from some donors may be more effective against certain classes of cancers or even against specific types of cancer. In one embodiment, a method of selecting γδ-T cells from the bank of γδ-T cells for use in treating cancer in a subject is provided. The selection method comprises selecting γδ-T cells from a donor with a specific HLA type that is a full mismatch with the HLA of the subject. In some embodiments, the HLA type of the selected γδ-T cells is a full HLA mismatch as compared to the HLA of any of the previous donors used for the same subject. The selection method may also comprise selecting γδ-T cells that are highly effective against the type of cancer that is being treated.

### C. Monoclonal Antibody Therapy

In some embodiments, the donor-derived allogeneic γδ-T cells are administered as a combination therapy comprising a therapeutically effective amount of at least one monoclonal antibody therapy. Various antibodies can be used in the methods of treating cancer provided herein. The various antibodies disclosed herein and for use in the methods provided herein can be produced using any antibody production method known to those of skill in the art.

The term "antibody" is used in the broadest sense and covers fully assembled antibodies, antibody fragments that can bind antigen (*e.g.*, Fab, F(ab')₂, Fv, single chain antibodies, diabodies, antibody chimeras, hybrid antibodies, bispecific antibodies, humanized antibodies, and the like), and recombinant peptides comprising the forgoing.

"Antibody fragments" comprise a portion of an intact antibody, preferably the antigen-binding or variable region of the intact antibody. Examples of antibody fragments include Fab, F(ab')2, and Fv fragments; diabodies; linear antibodies (Zapata et al. (1995) Protein Eng. 10: 1057-1062); single-chain antibody molecules; and multispecific antibodies formed from antibody fragments.

In one embodiment, the antibody is a monoclonal antibody. By "monoclonal antibody" is intended an antibody obtained from a population of substantially homogeneous antibodies, that is, the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, such as those produced by a clonal population of B-cells, and is not to be construed as requiring production of the antibody by any particular method.

For example, the monoclonal antibodies to be used in accordance with the methods provided herein may be made by the hybridoma method first described by Kohler et al. (1975) Nature 256:495, or a modification thereof. Typically, a mouse is immunized with a solution containing an antigen. Immunization can be performed by mixing or emulsifying the antigen-containing solution in saline, preferably in an adjuvant such as Freund's complete adjuvant, and injecting the mixture or emulsion parenterally. Any method of immunization known in the art may be used to obtain the monoclonal antibodies. After immunization of the animal, the spleen (and optionally, several large lymph nodes) are removed and dissociated into single cells. The spleen cells may be screened by applying a cell suspension to a plate or well coated with the antigen of interest. The B-cells expressing membrane bound immunoglobulin specific for the antigen bind to the plate and are not rinsed away. Resulting B-cells, or all dissociated spleen cells, are then induced to fuse with myeloma cells to form hybridomas, and are cultured in a selective medium. The resulting cells are plated by serial dilution and are assayed for the production of antibodies that specifically bind the antigen of interest (and that do not bind to unrelated antigens). The selected monoclonal antibody (mAb)-secreting hybridomas are then cultured either *in vitro* (e.g., in tissue culture bottles or hollow fiber reactors), or *in vivo* (as ascites in mice).

Alternatively, the monoclonal antibodies may be made by recombinant DNA methods (see, *e.g.,* U.S. Patent No. 4,816,567). The monoclonal antibodies may also be isolated from phage antibody libraries using the techniques described in, for example, Clackson et al. (1991) Nature 352:624-628; Marks et al. (1991) J. Mol. Biol. 222:581-597; and U.S. Patent No. 5,514,548.

By "epitope" is intended the part of an antigenic molecule to which an antibody is produced and to which the antibody will bind. Epitopes can comprise linear amino acid residues (*i.e.,* residues within the epitope are arranged sequentially one after another in a linear fashion), nonlinear amino acid residues (referred to herein as "nonlinear epitopes"-these epitopes are not arranged sequentially), or both linear and nonlinear amino acid residues.

Additionally, the term "antibody" as used herein encompasses chimeric and humanized monoclonal antibodies. By "chimeric" antibodies is intended antibodies that are most preferably derived using recombinant deoxyribonucleic acid techniques and which comprise both human (including immunologically "related" species, *e.g*., chimpanzee) and non-human components. Thus, the constant region of the chimeric antibody is substantially identical to the constant region of a natural human antibody; the variable region of the chimeric antibody is most preferably derived from a non-human source and has the desired antigenic specificity. The non-human source can be any vertebrate source that can be used to generate antibodies to a tumor antigen or material comprising a polypeptide of a tumor antigen. Such non-human sources include, but are not limited to, rodents (*e.g.,* rabbit, rat, mouse, *etc*.; see, *e.g.,* U.S. Patent No. 4,816,567) and non-human primates (*e.g.,* Old World Monkeys, Apes, *etc*.; see, *e.g.,* U.S. Patent Nos. 5,750,105 and 5,756,096).

In some embodiments, the monoclonal antibody therapy comprises a humanized antibody. By "humanized" is intended forms of antibodies that contain minimal sequence derived from non-human immunoglobulin sequences. Accordingly, such "humanized" antibodies may include antibodies wherein substantially less than an intact human variable domain has been substituted by the corresponding sequence from a non-human species.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1, and IgA2. The heavy-chain constant domains that correspond to the different classes of immunoglobulins are called alpha, delta, epsilon, gamma, and mu, respectively. The subunit structures and three-dimensional configurations of different classes of immunoglobulins are well known. Different isotypes have different effector functions. For example, IgG1 and IgG3 isotypes have ADCC (antibody dependent cell-mediated cytotoxicity) activity.

A monoclonal antibody for use in the methods provided herein can be of any of the various antibody classes. In one embodiment, the monoclonal antibody is an IgG class antibody. In other embodiments, the monoclonal antibody can be of the IgM, IgE, IgD, or IgA class. In specific embodiments, the antibody is an isotype of IgG, such as, IgG1, IgG2, IgG3 or IgG4.

The monoclonal antibodies for use in the methods provided herein can recognize any antigen. In some embodiments, the monoclonal antibody recognizes a tumor antigen. Any known therapeutic monoclonal antibody can be used in the combination therapy provided herein.

For example, therapeutic antibodies against CD20 are known in the art for treating various types of B-cell malignancies and include, for example, Rituximab (Rituxan) (see, for example, Weiner, GJ. (2010) Semin Hematol. 47(2):115-123). In one embodiment, the monoclonal antibody comprises a CD20 antibody. In a specific embodiment, the monoclonal antibody therapy comprises Rituximab.

In another non-limiting example, therapeutic antibodies against HER-2, such as Herceptin (trastuzumab) are known in the art and are used in the treatment of breast cancer (see, for example, Capietto AH, et al. (2011) J Immunol. 187(2): 1031-8). In one embodiment, the monoclonal antibody comprises a HER-2 antibody. In a specific embodiment, the monoclonal antibody therapy is Herceptin (trastuzumab).

Another non-limiting example includes checkpoint inhibitor antibodies, including, but not limited to, nivolumab (Opdivo), pembrolizumab (Keytruda) or ipilimumab (Yervoy).

In some embodiments, more than one monoclonal antibody therapy can be administered. In a specific embodiment, two or more monoclonal antibody therapies can be administered to a subject.

In another embodiment, the therapeutic antibody may be a tribody. A tribody refers to a multifunctional antibody derivative. A tribody can comprise antigen binding domains and/or effector domains, including, but not limited to, immunotoxins, immunocytokines, enzyme functions, or any functional domain. In one embodiment, the tribody comprises one or more effector domains that enhance the anti-tumor activity of γδ-T cells. In another embodiment, the tribody comprises one or more antigen binding domains specific for one or more tumor antigens. In a specific embodiment, the tribody comprises one or more antigen binding domains that bind to HER2 and a CD16 binding domain, including, but not limited to, the [(HER2)2xCD16] tribody described in Oberg et al. (2018) Front. Immunol. 9:814*.*

### D. Combination Therapy

The methods provided herein may comprise a combination therapy of donor-derived allogeneic γδ-T cells and one or more therapeutic agents. The term "combination" or "combination therapy" is used herein in its broadest sense and means that a subject is treated with at least two therapeutic regimens. The timing of administration of the different therapeutic regimens can be varied so long as the beneficial effects of the combination of these therapeutic regimens are achieved. Treatment with donor-derived allogeneic γδ-T cells in combination with one or more therapeutic agents can be simultaneous (concurrent), consecutive (sequential), or a combination thereof. Therefore, a subject undergoing combination therapy can receive both donor-derived allogeneic γδ-T cells and one or more therapeutic agents at the same time (i.e., simultaneously), or at different times (i.e. sequentially, in either order, on the same day, or on different days), as long as the therapeutic effect of the combination of both is caused in the subject undergoing therapy. Where the donor-derived allogeneic γδ-T cells or one or more therapeutic agents are administered simultaneously, they can be administered as separate pharmaceutical compositions, each comprising either donor-derived allogeneic γδ-T cells or therapeutic agent, or can be administered as a single pharmaceutical composition comprising both of these agents. In some embodiments, one or more therapeutic agents are administered as a pre-treatment prior to administration of the donor-derived allogeneic γδ-T cells. The one or more therapeutic agents may be administered to the subject at least 2 hours, 4 hours, 6 hours, 8 hours, 12 hours, 24 hours, 48 hours, 72 hours, or more prior to the administration of the donor-derived allogeneic γδ-T cells.

The combination therapy provided herein can also be achieved intermittently. By "intermittent combination therapy" is intended a period of combination therapy with donor-derived allogeneic γδ-T cells and one or more therapeutic agents, followed by a time period of discontinuance, which is then followed by another period of combination therapy with donor-derived allogeneic γδ-T cells and one or more therapeutic agents, and so forth.

In the methods provided herein, the donor-derived allogeneic γδ-T cells may be combined with one or more therapeutic agents to potentiate the in vivo killing activity and/or homing ability of the γδ-T cells. As such, tumors, including tumors that are relatively resistant to treatment, can be sensitized to killing by γδ-T cells. γδ-T cell sensitizing agents include, but are not limited to, chemotherapeutic agents, including etoposide, cisplatin, doxorubicin, 5-Fluorouracil, vincristine, bortezomib and oxaliplatin; small molecules, including ibrutinib; therapeutic antibodies, including rituximab, trastuzumab, nivolumab, pembrolizumab, and ipilimumab; or amino-bisphosphonates, including zoledronic acid. In one embodiment, the methods provided herein further comprise administering to the subject one or more of etoposide, cisplatin, doxorubicin, 5-Fluorouracil, vincristine, bortezomib, oxaliplatin or ibrutinib.

The methods provided herein include pre-treating the subject with an amino-bisphosphonate prior to administration of the donor-derived allogeneic γδ-T cells. As described elsewhere herein, amino-bisphosphonates (such as zoledronic acid) can act indirectly on γδ-T cells by causing bystander cells to release isopentenyl pyrophosphate (IPP). This happens since in bystander cells, zoledronic acid inhibits the enzyme farnesyl pyrophosphate synthase (FPPS), a critical enzyme in the mevalonate biosynthetic pathway. Disruption of the pathway results in the accumulation of IPP which is eventually released from cells which then in turn, stimulates γδ-T cells. Tumor cells exposed to amino-bisphosphonates (such as zoledronic acid) also can be made to release IPP and related phosphoantigens. As tumor cells already preferentially produce IPP as a result of their dysregulated state, then the effect of an amino-bisphosphonate is even greater. It is this enhanced release of IPP by tumor cells first exposed to an amino-bisphosphonate that makes these cells more sensitive to killing by γδ-T cells. In addition, this IPP can serve as a chemo attractant to γδ-T cells, making them even more potent in vivo. According to the claimed invention, the method of treating cancer comprises administering zoledronic acid to the subject prior to the administration of the donor-derived allogeneic γδ-T cells.

The donor-derived allogeneic γδ-T cells may also be administered as a combination therapy with other cellular therapies in order to potentiate anti-tumor activity. The donor-derived allogeneic γδ-T cells may be administered concurrently or sequentially with any number of other cell types, including, but not limited to, γδ-T cells of the Vδ1 variety, or any γδ-T cell subset that is non-Vδ2; NK cells; or CAR-T cells (either αβ-T cell or γδ-T cell derived). In one embodiment, the methods provided herein further comprise administering one or more additional cellular therapies. In some methods, the one or more additional cellular therapies are from the same allogeneic donor as the γδ-T cells.

As described elsewhere herein, the methods provided herein may comprise a combination therapy of donor-derived allogeneic γδ-T cells and a monoclonal antibody therapy that allows for the targeted killing of tumor cells in a subject with cancer. In such methods, a monoclonal antibody against a surface tumor antigen can redirect the donor-derived allogeneic γδ-T cells to tumors. Although γδ-T cells have innate anti-tumor activity independent of ADCC, activation of γδ-T cells with at least one γδ-T cell expansion compound (i.e. any of the various γδ-T cell expansion compounds provided herein) may upregulate the expression of Fc receptors thereby making the γδ-T cells more likely to bind to the Fc portion of an antibody. As such, the combination therapy may enhance ADCC mediated by therapeutic antibodies by specifically directing the γδ-T cells to tumor targets. See, for example, Gertner-Dardenne J, et al. (2009) Blood. 113:4875-4884; and Capietto AH, et al. (2011) J. Immunol. 187(2):1031-8. In one embodiment, the methods provided herein further comprise administering at least one dose of a therapeutically effective amount of at least one monoclonal antibody therapy to the subject. In specific embodiments, the monoclonal antibody recognizes a tumor antigen.

In one embodiment of the first aspect of the invention, a method for treating cancer in a subject is provided, the method comprising: administering at least one lymphodepletion treatment to the subject; and, subsequently administering a combination therapy of the at least one dose of donor-derived allogeneic γδ-T cells and at least one dose of a therapeutically effective amount of at least one monoclonal antibody therapy to the subject, wherein the γδ-T cells are expanded ex vivo by culturing the γδ-T cells with the at least one γδ-T cell expansion compound prior to administration of the γδ-T cells to the subject; wherein the combination therapy results in an antitumor response in the subject. In another embodiment of the first aspect of the invention, a method for treating cancer in a subject is provided, the method comprising: administering a Hematopoietic Stem Cell (HSC) transplant to the subject; and subsequently administering a combination therapy of the at least one dose of donor-derived allogeneic γδ-T cells and at least one dose of a therapeutically effective amount of at least one monoclonal antibody therapy to the subject, wherein the γδ-T cells are expanded ex vivo by culturing the γδ-T cells with the at least one γδ-T cell expansion compound prior to administration of the γδ-T cells to the subject; wherein the combination therapy results in an antitumor response in the subject.

In some embodiments, the combination therapy of donor-derived allogeneic γδ-T cells and monoclonal antibody therapy, results in an antitumor response that is greater than the anti-tumor response that would be observed with donor-derived allogeneic γδ-T cells therapy or monoclonal antibody therapy alone. By "greater than" is meant that the combination therapy results in a statistically significant increase in antitumor response as compared to either γδ-T cell therapy or monoclonal antibody therapy alone. The antitumor response of the combination therapy can be any statistically significant increase of at least 2%, 5%, 10%, 20%, 30%, 40 %, 50%, 60%, 70%, 80%, 90%, 100%, 200% or more as compared to the antitumor response of either γδ-T cell therapy or monoclonal antibody therapy alone.

In other embodiments, the combination therapy of at least one dose of donor-derived allogeneic γδ-T cells and at least one dose of a therapeutically effective amount of at least one monoclonal antibody therapy induces antibody-dependent cell-mediated cytotoxicity (ADCC).

In a specific embodiment, the donor-derived allogeneic γδ-T cells and therapeutically effective amount of at least one monoclonal antibody therapy are administered to the subject simultaneously. In another specific embodiment, the donor-derived allogeneic γδ-T cells and therapeutically effective amount of at least one monoclonal antibody therapy are administered to the subject at different times.

According to the claimed invention, the subject is a human.

The ex vivo expanded donor-derived allogeneic γδ-T cells provided herein may also be precoated with monoclonal antibodies prior to administering the γδ-T cells to a subject. In such a method, the γδ-T cells would be cultured with the antibodies in order to allow the Fc receptors on the γδ-T cells to bind to the Fc portion of the antibodies for subsequent administration to a subject. The γδ-T cells and antibodies may be cultured for at least 1 hr, at least 2 hr, at least 4 hr, at least 6 hr, at least 8 hr, at least 10 hr, at least 12 hr, at least 18 hr, at least 24 hr, at least 48 hr or more as long as the γδ-T cells have bound the antibodies. Assays to measure binding of the antibodies to the Fc receptors on the γδ-T cells are known in the art and include, for example, flow cytometry, radioligand binding assays, fluorometric binding assays or colorimetric binding assays.

In some embodiments, multiple administrations of antibody coated γδ-T cells may be administered to a subject in the course of treatment for a cancer. In other embodiments, the subject receiving the antibody coated γδ-T cells may also receive administrations of donor-derived allogeneic γδ-T cells, monoclonal antibody therapy and/or the combination of donor-derived allogeneic γδ-T cells and monoclonal antibody therapy as provided herein.

### III. Methods of Administration

The methods of treating cancer provided herein can encompass administration of treatment via any parenteral route, including, but not limited, to intramuscular, intraperitoneal, intravenous, and the like.

Further, as used herein "pharmaceutically acceptable carriers" are well known to those skilled in the art and include, but are not limited to, 0.01-0.1 M, or 0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, collating agents, inert gases and the like.

Controlled or sustained release compositions include formulation in lipophilic depots (e.g. fatty acids, waxes, oils). Also comprehended herein are particulate compositions coated with polymers (e.g. poloxamers or poloxamines) and the compound coupled to antibodies directed against tissue-specific receptors, ligands or antigens or coupled to ligands of tissue-specific receptors. Other embodiments of the compositions presented herein incorporate particulate forms protective coatings, protease inhibitors or permeation enhancers for various routes of administration, including parenteral, pulmonary, nasal and oral.

When administered, compounds are often cleared rapidly from mucosal surfaces or the circulation and may therefore elicit relatively short-lived pharmacological activity. Consequently, frequent administrations of relatively large doses of bioactive compounds may be required to sustain therapeutic efficacy. Compounds modified by the covalent attachment of water-soluble polymers such as polyethylene glycol, copolymers of polyethylene glycol and polypropylene glycol, carboxymethyl cellulose, dextran, polyvinyl alcohol, polyvinylpyrrolidone or polyproline are known to exhibit substantially longer half-lives in blood following intravenous injection than do the corresponding unmodified compounds (Abuchowski *et al.,* 1981; Newmark *et al.,* 1982; and Katre *et al.,* 1987). Such modifications may also increase the compound's solubility in aqueous solution, eliminate aggregation, enhance the physical and chemical stability of the compound, and greatly reduce the immunogenicity and reactivity of the compound. As a result, the desired *in vivo* biological activity may be achieved by the administration of such polymer-compound abducts less frequently or in lower doses than with the unmodified compound.

*Dosages for antibody therapy.* The sufficient amount may include but is not limited to from about 1 µg/kg to about 100 µg/kg, from about 100 µg/kg to about 1 mg/kg, from about 1 mg/kg to about 10 mg/kg, about 10 mg/kg to about 100 mg/kg, from about 100 mg/kg to about 500 mg/kg or from about 500 mg/kg to about 1000 mg/kg. The amount may be 10 mg/kg. The pharmaceutically acceptable form of the composition includes a pharmaceutically acceptable carrier.

The preparation of therapeutic compositions which contain an active component is well understood in the art. Typically, such compositions are prepared as an aerosol of the polypeptide delivered to the nasopharynx or as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, pH buffering agents which enhance the effectiveness of the active ingredient.

An active component can be formulated into the therapeutic composition as neutralized pharmaceutically acceptable salt forms. Pharmaceutically acceptable salts include the acid addition salts (formed with the free amino groups of the polypeptide) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The component or components of a therapeutic composition provided herein may be introduced parenterally, transmucosally, e.g., orally, nasally, pulmonarily, or rectally, or transdermally. Preferably, administration is parenteral, e.g., via intravenous injection, and also including, but is not limited to, intra-arteriole, intramuscular, intradermal, subcutaneous, intraperitoneal, intraventricular, and intracranial administration. The term "unit dose" when used in reference to a therapeutic composition provided herein refers to physically discrete units suitable as unitary dosage for humans, each unit containing a predetermined quantity of active material calculated to produce the desired therapeutic effect in association with the required diluent; *i.e.,* carrier, or vehicle.

In another embodiment, the active compound can be delivered in a vesicle, in particular a liposome (see Langer (1990) Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid).

In yet another embodiment, the therapeutic compound can be delivered in a controlled release system. For example, the therapeutic composition may be administered using intravenous infusion, an implantable osmotic pump, a transdermal patch, liposomes, or other modes of administration. In one embodiment, a pump may be used (see Langer, supra; Sefton (1987) CRC Crit. Ref. Biomed. Eng. 14:201; Buchwald et al. (1980) Surgery 88:507; Saudek et al. (1989) N. Engl. J. Med. 321:574). In another embodiment, polymeric materials can be used (see Medical Applications of Controlled Release, Langer and Wise (eds.), CRC Pres., Boca Raton, Florida (1974*);* Controlled Drug Bioavailability, Drug Product Design and Performance, Smolen and Ball (eds.), Wiley, New York (1984); Ranger and Peppas (1983) J. Macromol. Sci. Rev. Macromol. Chem. 23:61; see also Levy et al. (1985) Science 228:190; During et al. (1989) Ann. Neurol. 25:351; Howard et al. (1989) J. Neurosurg. 71:105). In yet another embodiment, a controlled release system can be placed in proximity of the therapeutic target, i.e., a tumor, thus requiring only a fraction of the systemic dose (see, e.g., Goodson, in Medical Applications of Controlled Release, supra, vol. 2, pp. 115-138 (1984)). Other controlled release systems are discussed in the review by Langer (1990) Science 249:1527-1533.

A subject in whom administration of an active component as set forth above is an effective therapeutic regimen for a cancer is a human.

In the therapeutic methods and compositions provided herein, a therapeutically effective dosage of the active component is provided. A therapeutically effective dosage can be determined by the ordinary skilled medical worker based on patient characteristics (age, weight, sex, condition, complications, other diseases, etc.), as is well known in the art. Furthermore, as further routine studies are conducted, more specific information will emerge regarding appropriate dosage levels for treatment of various conditions in various patients, and the ordinary skilled worker, considering the therapeutic context, age and general health of the recipient, is able to ascertain proper dosing. Generally, for intravenous injection or infusion, dosage may be lower than for intraperitoneal, intramuscular, or other route of administration. The dosing schedule may vary, depending on the circulation half-life, and the formulation used. The compositions are administered in a manner compatible with the dosage formulation in the therapeutically effective amount. Precise amounts of active ingredient required to be administered depend on the judgment of the practitioner and are peculiar to each individual. However, suitable dosages may range from about 0.1 to 20, preferably about 0.5 to about 10, and more preferably one to several, milligrams of active ingredient per kilogram body weight of individual per day and depend on the route of administration. Suitable regimes for initial administration and booster shots are also variable, but are typified by an initial administration followed by repeated doses at one or more hour intervals by a subsequent injection or other administration. Alternatively, continuous intravenous infusion sufficient to maintain concentrations of ten nanomolar to ten micromolar in the blood are contemplated.

*Administration with other compounds.* For treatment of cancer, one may administer the present active component in conjunction with one or more pharmaceutical compositions used for treating cancer, including but not limited to chemotherapeutic agents. Administration may be simultaneous (for example, administration of a mixture of the present active component and a chemotherapeutic agent), or may be *in seriatim.*

Those skilled in the art recognize that the methods of therapy disclosed herein may be used before, after, or concurrently with other forms of oncotherapy. Such oncotherapy can include chemotherapy regimens or radiation treatment.

Also contemplated are dry powder formulations comprising at least one protein provided herein and another therapeutically effective drug, such as a chemotherapeutic agent.

Contemplated for use herein are oral solid dosage forms, which are described generally in Remington's Pharmaceutical Sciences, 18th Ed. 1990 (Mack Publishing Co. Easton PA 18042) at Chapter 89. Solid dosage forms include tablets, capsules, pills, troches or lozenges, cachets or pellets. Also, liposomal or proteinoid encapsulation may be used to formulate the present compositions (as, for example, proteinoid microspheres reported in U.S. Patent No. 4,925,673). Liposomal encapsulation may be used and the liposomes may be derivatized with various polymers (*e.g.,* U.S. Patent No. 5,013,556). A description of possible solid dosage forms for the therapeutic is given by Marshall, K. In: Modern Pharmaceutics Edited by G.S. Banker and C.T. Rhodes Chapter 10, 1979. In general, the formulation will include the component or components (or chemically modified forms thereof) and inert ingredients which allow for protection against the stomach environment, and release of the biologically active material in the intestine.

Also specifically contemplated are oral dosage forms of the above derivatized component or components. The component or components may be chemically modified so that oral delivery of the derivative is efficacious. Generally, the chemical modification contemplated is the attachment of at least one moiety to the component molecule itself, where the moiety permits (a) inhibition of proteolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis (1981) "Soluble Polymer-Enzyme Abducts" In: Enzymes as Drugs, Hocenberg and Roberts, eds., Wiley-Interscience, New York, NY, pp. 367-383; Newmark, et al. (1982) J. Appl. Biochem. 4:185-189. Other polymers that could be used are poly-1,3-dioxolane and poly-1,3,6-tioxocane. Preferred for pharmaceutical usage, as indicated above, are polyethylene glycol moieties.

For the component (or derivative) the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the protein (or derivative) or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and Shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic i.e. powder; for liquid forms, a soft gelatin shell may be used. The shell material of cachets could be thick starch or other edible paper. For pills, lozenges, molded tablets or tablet triturates, moist massing techniques can be used.

The peptide therapeutic can be included in the formulation as fine multiparticulates in the form of granules or pellets of particle size about 1mm. The formulation of the material for capsule administration could also be as a powder, lightly compressed plugs or even as tablets. The therapeutic could be prepared by compression.

Colorants and flavoring agents may all be included. For example, the protein (or derivative) may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, a-lactose, anhydrous lactose, cellulose, sucrose, modified dextran and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants. Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An antifrictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents might be used and could include benzalkonium chloride or benzethomium chloride. The list of potential nonionic detergents that could be included in the formulation as surfactants are lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the protein or derivative either alone or as a mixture in different ratios.

Additives which potentially enhance uptake of the protein (or derivative) are for instance the fatty acids oleic acid, linoleic acid and linolenic acid.

*Administration of donor-derived γδ*-*T cells.* Generally, compositions of γδ-T cells are prepared in the form suited for the route of administration thereof, for example in the form of injections, transfusions or like liquids or solutions. The liquid or solution forms, inclusive of injections, can be prepared in the same manner as in preparing various conventional pharmaceutical preparations as described above. The carrier to be used may be any of various pharmaceutically acceptable carriers (diluents) well known in the art. Non-limiting examples thereof are PBS and RPMI 1640. In preparing the above-mentioned liquid or solution forms, various technologies currently in general use in preparing various transfusions can be used. The γδ-T cell compositions may be prepared just prior to use. The γδ-T cell compositions are administered at respective predetermined doses via a predetermined route(s) of administration according to the method described herein. For example, the number of cells for infusion into a subject can be administered in the range from about 1 x 10³ to about 1 x 10¹⁰, from about 1 x 10⁴ to about 1 x 10¹⁰, from about 1 x 10⁵ to about 1 x 10¹⁰, form about 1 x 10⁶ to about 1 x 10¹⁰, form about 1 x 10⁷ to about 1 x 10¹⁰, form about 1 x 10⁸ to about 1 x 10¹⁰, from about 1 x 10³ to about 1 x 10⁹, form about 1 x 10⁵ to about 1 x 10⁹, form about 1 x 10⁶ to about 1 x 10⁹, form about 1 x 10⁴ to about 1 x 10⁸, form about 1 x 10⁶ to about 1 x 10⁸, or form about 1 x 10⁵ to about 1 x 10⁷ per infusion.

It is recognized that the method of treatment may comprise a single administration of a therapeutically effective dose of the therapy or multiple administrations of a therapeutically effective dose of the therapy. Moreover, the treatment can be accomplished with varying doses as well as dosage regimens.

As used herein, the singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description.

The subject matter of the present disclosure is further illustrated by the following non-limiting examples.

### EXAMPLES

### Example 1 - Generation of ex vivo Expanded Human γδ-T cells using C-HDMAPP

### Starting Material: PBMC from a Healthy Donor

Peripheral blood mononuclear cells (PBMC) were isolated from peripheral blood obtained by venipuncture from a healthy individual. Fresh blood was collected using standard aseptic technique. PBMC were isolated utilizing a standard density gradient centrifugation method. PBMC were washed in PBS, then resuspended in complete RPMI-1640 supplemented with 10% characterized fetal bovine serum and PCN + streptomycin.

### Ex vivo Expansion of Human γδ-T cells

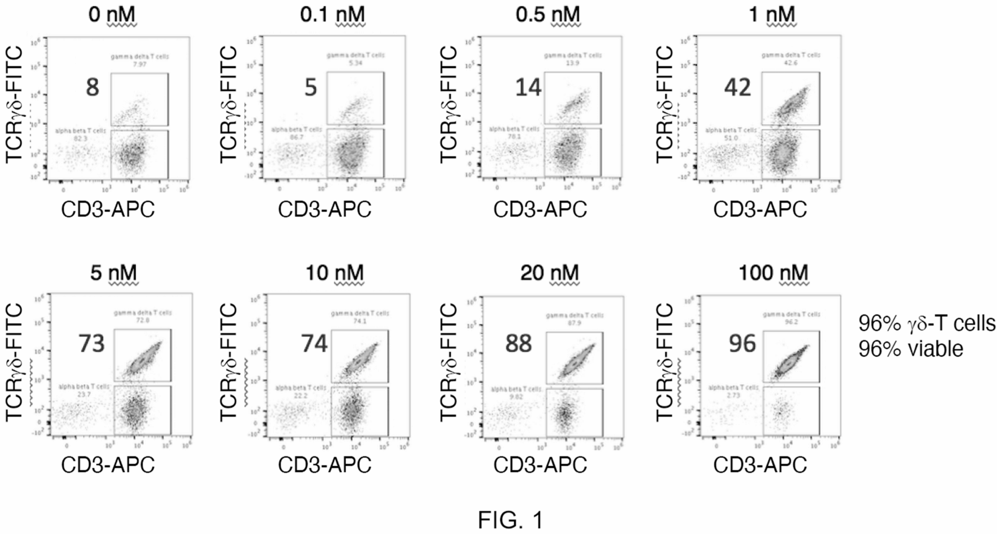

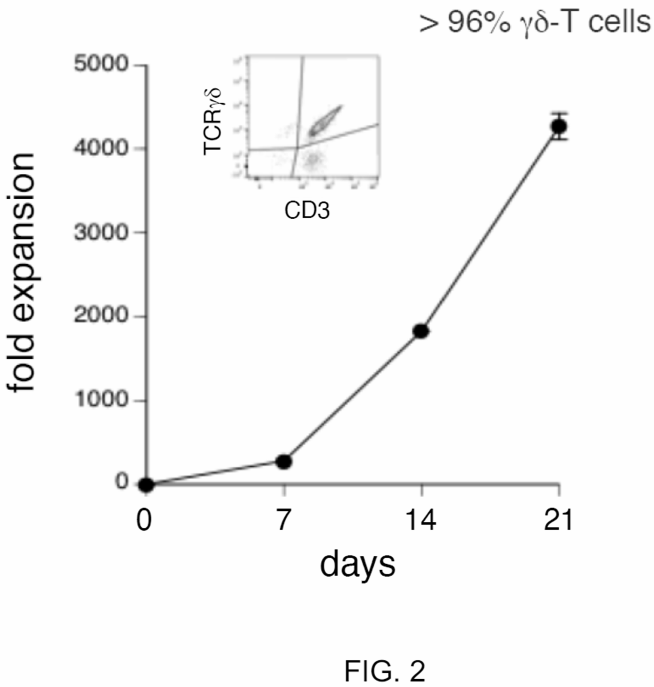

Cell cultures were initiated by seeding PBMC into tissue culture flasks to which recombinant human IL-2 was added. γδ-T cell expansion was initiated by the addition of C-HDMPP. Cultures were maintained at 37° C, 5% CO₂ in incubators situated within a BSL2 containment environment. Fresh media was added as needed to keep cell concentration at approximately 1 x 10⁶ cells/ml. Human IL-2 was added every 5 days. Cultures were maintained for 21 days at which time they were analyzed by flow cytometry. FIG. 1 shows that C-HDMPP used at nanomolar concentrations can be titrated to promote optimal γδ-T cell growth while avoiding overstimulation. At 100 nM C-HDMPP the cultures were ≥96% pure γδ-T cells with viability ≥96%. FIG. 2 demonstrates the fold expansion of the γδ-T cells over 21 days of culture with 100 nM C-HDMPP.

### Example 2- Cryopreservation

After 21 days, cell clusters in culture are gently disrupted, transferred to 50 ml conical tubes and pelleted by centrifugation. Supernatants are discarded and cell pellets are resuspended with complete RPMI then centrifuged again at 300 x g for 15 min at 20° C. Cell pellets are resuspended in cell culture media containing 10% DMSO. Cells are transferred to sterile cryovials in aliquots of 1 ml or 2 ml. Cryovials are placed in a controlled rate freezing device (-1°C/minute freezing container), which are transferred to a -80° C freezer and subsequently transferred to a liquid nitrogen storage system for storage in the liquid nitrogen vapor phase at -135° C.

### Thawing and Overnight Culture in IL-2 before Use

One day before use, the cryovials containing γδ-T cells are removed from liquid nitrogen storage and transported on dry ice to the BSL2 laboratory. The cells are partially defrosted in a 37° C water bath. Partially-thawed vials are filled with chilled culture media added dropwise. Contents of each vial are transferred dropwise into a 50 mL tube containing culture media. After centrifugation, cell pellets are resuspended at a concentration of 1 x 10⁶ cells/ml in complete RPMI (supplemented with 10% FBS) containing human IL-2. Cells are maintained in a BSL2 laboratory incubator at 37° C, 5% CO₂ for 24 hours. The next day, cells are evaluated by flow cytometry to confirm viability and purity. The cells are washed (to remove IL-2) and resuspended at a final concentration suitable for their intended use (in vitro study, in vivo xenograft, or human clinical use).

### Example 3 - Antitumor Activity of ex vivo Expanded Human γδ-T cells

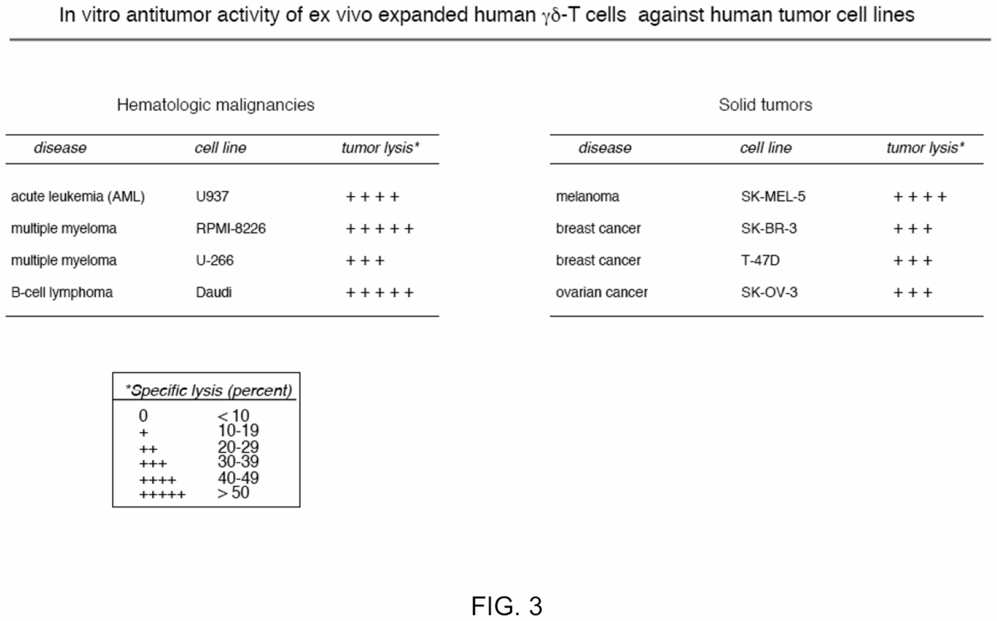

Human γδ-T cells were expanded using the methods described in Example 1. After 21 days in culture, cells were used as effector cells (killer cells) for in vitro cytotoxicity assays against selected tumor target cells. The indicated human tumor cell lines were each first labeled with carboxyfluorescein succinimidyl ester (CFSE) using standard methods. Effector cells and CFSE-labeled tumor cells where co-cultured for four hours at various effector to target (E:T) ratios. Upon completion of co-culture, propidium iodide was added to the cell mixture which was then analyzed by multicolor FACS. By gating only on CFSE-positive events (tumor cells), the proportion of tumor cells that were PI-positive (dead) was determined, allowing for the calculation of specific tumor cell death. The results in FIG. 3 are shown as tumor cell lysis at the E:T ratio of 25: 1. FIG. 3 demonstrates that the expanded γδ-T cells have anti-tumor activity against various tumor cells, including hematologic malignancies and cells from solid tumors.

### Example 4 - Sensitization of Tumor Cells to Killing by γδ-T cells

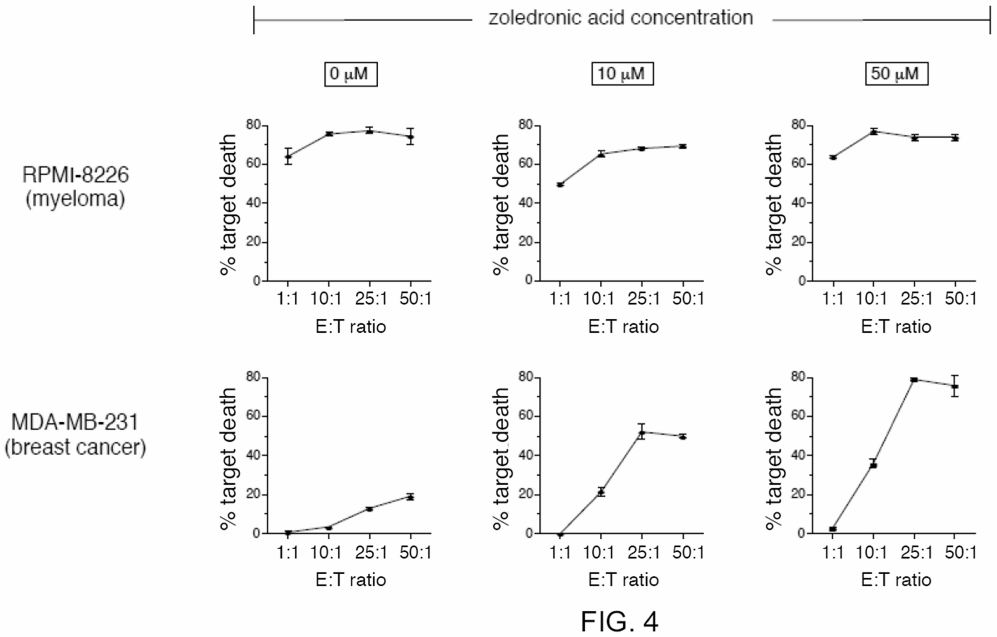

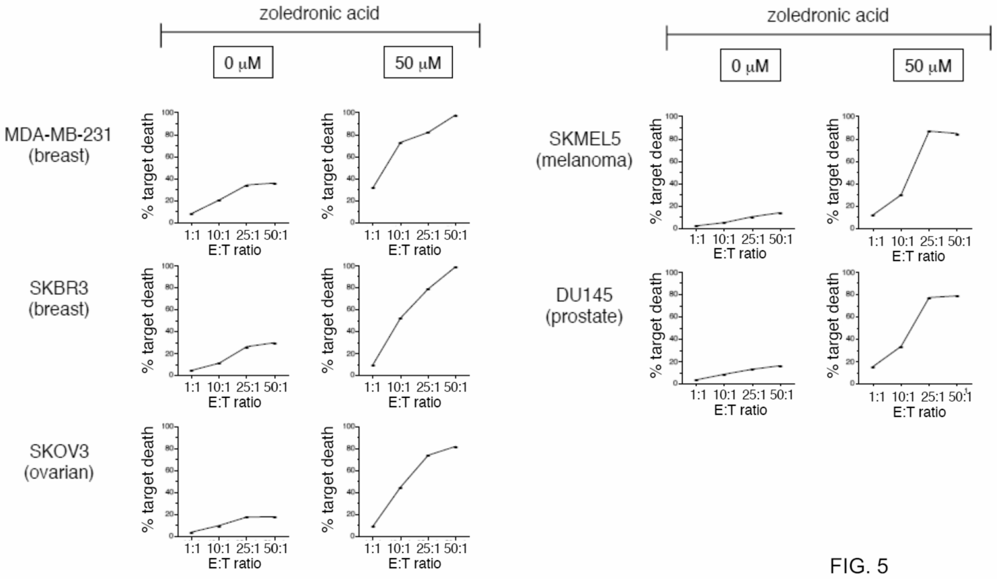

Human γδ-T cells were expanded using the methods described in Example 1. The expanded γδ-T cells were used as effector cells (killer cells) for in vitro cytotoxicity assays against selected tumor target cells. Twenty-four hours prior to killing assays, the human tumor cell lines were each cultured in the presence of zoledronic acid (ZOL) at various concentrations (0 µM; 10 µM or 50 µM). Cells were carefully washed before use in killing assays. The effector cells and tumor cells were then co-cultured for four hours at various effector to target (E:T) ratios. Using either FACS-based methods (described in Example 3) or a commercially available LDH-release assay (Promega LDH-Glo), specific tumor cell death was calculated. The results in FIG. 4 and FIG. 5 are shown as tumor cell lysis at the indicated E:T ratios. These results demonstrate that exposure to low concentrations of zoledronic acid renders various tumor cells more sensitive to killing by γδ-T cells. This includes tumor cells that are relatively resistant to γδ-T cells.

## Claims

1. Donor-derived allogeneic γδ-T cells for use in a method for treating cancer in a human subject, the method comprising:
(a) administering at least one lymphodepletion treatment or a Hematopoietic Stem Cell (HSC) transplant to the subject;
(b) administering zoledronic acid to the subject; and,
(c) subsequently administering at least one dose of the donor-derived allogeneic γδ-T cells to the subject, wherein the γδ-T cells are expanded ex vivo by culturing the γδ-T cells with at least one γδ-T cell expansion compound prior to administration of the γδ-T cells to the subject;
wherein the at least one γδ-T cell expansion compound comprises C-HDMAPP; wherein the treatment results in an antitumor response in the subject.

2. The donor-derived allogeneic γδ-T cells for the use according to claim 1, wherein the lymphodepletion treatment comprises administering one or more chemotherapeutic agents or low dose total body irradiation.

3. The donor-derived allogeneic γδ-T cells for the use according to claim 2, wherein the one or more chemotherapeutic agents comprise cyclophosphamide, fludarabine or melphalan.

4. The donor-derived allogeneic γδ-T cells for the use according to any one of claims 1-3, further comprising administering to the subject:
a) one or more of etoposide, cisplatin, doxorubicin, 5-Fluorouracil, vincristine, bortezomib, oxaliplatin or ibrutinib;
b) a therapeutically effective amount of at least one monoclonal antibody therapy; and/or
c) one or more additional cellular therapies.

5. The donor-derived allogeneic γδ-T cells for the use according to claim 4, wherein the monoclonal antibody recognizes a tumor antigen.

6. The donor-derived allogeneic γδ-T cells for the use according to claim 4, wherein the one or more additional cellular therapies are from the same allogeneic donor as the γδ-T cells.

7. The donor-derived allogeneic γδ-T cells for the use according to any one of claims 1-6, further comprising culturing the γδ-T cells with interleukin-2.

8. The donor-derived allogeneic γδ-T cells for the use according to any one of claims 1-7, further comprising culturing the γδ-T cells with one or more checkpoint inhibitors prior to administration of the γδ-T cells to the subject.

9. The donor-derived allogeneic γδ-T cells for the use according to claim 8, wherein the one or more checkpoint inhibitors comprises an anti-PD-1 antibody, an anti-PDL1 antibody, an anti-TIM-3 antibody, an anti-LAG3 antibody, an anti-galectin-9 antibody, an anti-IDO antibody and/or an anti-VISTA antibody.

10. The donor-derived allogeneic γδ-T cells for the use according to any one of claims 1-9, further comprising culturing the γδ-T cells with one or more of IL-15, an anti-CD277 antibody, an anti-TGF-beta antibody, a prostaglandin E₂ inhibitor, adenosine, osteopontin, vitamin C and/or a hypomethylating agent prior to administration of the γδ-T cells to the subject.

11. The donor-derived allogeneic γδ-T cells for the use according to any one of claims 1-10, wherein the subject and the donor of the γδ-T cells have a full human leukocyte antigen (HLA) mismatch.

12. The donor-derived allogeneic γδ-T cells for the use according to any one of claims 1-11, further comprising repeating steps (a) and (c) one or more times, wherein the donor-derived allogeneic γδ-T cells for each subsequent administration are from a different donor having a full HLA mismatch as compared to the subject and the previous donor(s).

## Patentansprüche

1. Von einem Spender stammende allogene γδ-T-Zellen für die Anwendung in einem Verfahren zum Behandeln von Krebs bei einer menschlichen Person, wobei das Verfahren umfasst
(a) Verabreichen von mindestens einer Lymphdepletionsbehandlung oder einer Transplantation hämatopoetischer Stammzellen (HSC) für den Patienten;
(b) Verabreichen von Zoledronsäure an den Patienten; und
(c) anschließendes Verabreichen von mindestens einer Dosis von den von dem Spender stammenden allogenen γδ-T-Zellen an den Patienten, wobei die γδ-T-Zellen ex vivo vermehrt werden, indem die γδ-T-Zellen mit mindestens einer Verbindung zur γδ-T-Zellvermehrung kultiviert werden, bevor die Verabreichung der γδ-T-Zellen an den Patienten erfolgt;
wobei die mindestens eine Verbindung zur γδ-T-Zellvermehrung C-HDMAPP umfasst;
wobei die Behandlungsergebnisse zu einer Antitumor-Reaktion bei dem Patienten führen.

2. Von einem Spender stammenden allogene γδ-T-Zellen für die Anwendung nach Anspruch 1, wobei die Lymphdepletionsbehandlung ein Verabreichen von einem oder mehreren chemotherapeutischen Wirkstoffen oder von einer niedrigen Dosis einer Gesamtkörperbestrahlung umfasst.

3. Von einem Spender stammende allogene γδ-T-Zellen für die Anwendung nach Anspruch 2, wobei der eine oder die mehreren chemotherapeutischen Wirkstoffe Cyclophosphamid, Fludarabin oder Melphalan umfassen.

4. Von einem Spender stammende allogene γδ-T-Zellen für die Anwendung nach einem der Ansprüche 1 bis 3, wobei die Behandlung ferner umfasst, dem Patienten Folgendes zu verabreichen und zu verordnen:
a) eine oder mehrere Einheiten von Etoposid, Cisplatin, Doxorubicin, 5-Fluorouracil, Vincristin, Bortezomib, Oxaliplatin oder Ibrutinib;
(b) eine therapeutisch wirksame Menge von mindestens einer monoklonalen Antikörpertherapie; und/oder
(c) eine oder mehrere zusätzliche zelluläre Therapien.

5. Von einem Spender stammende allogene γδ-T-Zellen für die Anwendung nach Anspruch 4, wobei der monoklonale Antikörper ein Tumorantigen erkennt.

6. Von einem Spender stammende allogene γδ-T-Zellen für die Anwendung nach Anspruch 4, wobei die eine oder die mehreren zusätzlichen zellulären Therapien von demselben allogenen Spender stammen wie die γδ-T-Zellen.

7. Von einem Spender stammende allogene γδ-T-Zellen für die Anwendung nach einem der Ansprüche 1 bis 6, wobei das Verfahren ferner ein Kultivieren der γδ-T-Zellen mit Interleukin-2 umfasst.

8. Von einem Spender stammende allogene γδ-T-Zellen für die Anwendung nach einem der Ansprüche 1 bis 7, wobei das Verfahren ferner ein Kultivieren der γδ-T-Zellen mit einem oder mehreren Checkpoint-Inhibitoren vor der Verabreichung der γδ-T-Zellen an den Patienten umfasst.

9. Von einem Spender stammende allogene γδ-T-Zellen für die Anwendung nach Anspruch 8, wobei der eine oder die mehreren Checkpoint-Inhibitoren einen anti-PD-1-Antikörper, einen anti-PDL1-Antikörper, einen anti-TIM-3-Antikörper, einen anti-LAG3-Antikörper, einen anti-Galektin-9-Antikörper, einen anti-IDO-Antikörper und/oder einen anti-VISTA-Antikörper umfassen.

10. Von einem Spender stammende allogene γδ-T-Zellen für die Anwendung nach einem der Ansprüche 1 bis 9, wobei das Verfahren ferner ein Kultivieren der γδ-T-Zellen mit einer oder mehreren Einheiten von IL-15, einen anti-CD277-Antikörper, einen anti-TGF-beta-Antikörper, einen Prostaglandin-E₂-Inhibitor, Adenosin, Osteopontin, Vitamin C und/oder einen hypomethylierenden Wirkstoff vor der Verabreichung der γδ-T-Zellen an den Patienten umfasst.

11. Von einem Spender stammende allogene γδ-T-Zellen für die Anwendung nach einem der Ansprüche 1 bis 10, wobei der Patient und der Spender der γδ-T-Zellen eine vollständige menschliche Leukozyten-Antigen-(HLA)-Inkompatibilität aufweisen.

12. Von einem Spender stammende allogene γδ-T-Zellen für die Anwendung nach einem der Ansprüche 1 bis 11, wobei das Verfahren ferner ein Wiederholen der Schritte (a) und (c) für ein oder mehrere Male umfasst, wobei die von einem Spender stammenden allogenen γδ-T-Zellen für jede nachfolgende Verabreichung von einem unterschiedlichen Spender sind, der eine vollständige HLA-Inkompatibilität aufweist im Vergleich zu dem Patienten und zu dem(den) vorhergehenden Spender(n).

## Revendications

1. Lymphocytes T γδ allogènes dérivés d'un donneur destinés à être utilisés dans un procédé de traitement du cancer chez un sujet humain, le procédé comprenant:
(a) l'administration d'au moins un traitement de lymphodéplétion ou d'une greffe de cellules souches hématopoïétiques (HSC) au sujet;
(b) l'administration de l'acide zolendronique au sujet; et
(c) l'administration subséquente d'au moins une dose des lymphocytes T γδ dérivés d'un donneur au sujet, les lymphocytes T γδ étant étendus ex vivo en cultivant les lymphocytes T γδ avec au moins un composé d'extension de lymphocyte T γδ avant l'administration des lymphocytes T γδ au sujet;
l'au moins un composé d'extension de lymphocyte γδ comprenant du C-HDMAPP, le traitement résultant en une réponse anti-tumorale chez le sujet.

2. Lymphocytes T γδ allogènes dérivés d'un donneur destinés à être utilisés selon la revendication 1, dans lesquels le traitement de lymphodéplétion comprend l'administration d'un ou de plusieurs agents chimiothérapeutiques ou d'une irradiation du corps total à faible dose.

3. Lymphocytes T γδ allogènes dérivés d'un donneur destinés à être utilisés selon la revendication 2, dans lesquels l'un ou plusieurs agents chimiothérapeutiques comprennent le cyclophosphamide, la fludarabine ou le melphalan.

4. Lymphocytes T γδ allogènes dérivés d'un donneur destinés à être utilisés selon l'une quelconque des revendications 1 à 3, comprenant en outre l'administration au sujet:
a) d'un ou de plusieurs de l'étoposide, du cisplatine, de la doxorubicine, du 5-fluorouracile, de la vincristine, du bortézomib, de l'oxaliplatine ou de l'ibrutinib;
b) d'une quantité thérapeutiquement efficace d'au moins une thérapie d'anticorps monoclonal;
et/ou
c) d'une ou de plusieurs thérapies cellulaires supplémentaires.

5. Lymphocytes T γδ allogènes dérivés d'un donneur destinés à être utilisés selon la revendication 4, dans lesquels l'anticorps monoclonal reconnaît un antigène tumoral.

6. Lymphocytes T γδ allogènes dérivés d'un donneur destinés à être utilisés selon la revendication 4, dans lesquels les une ou plusieurs thérapies cellulaires supplémentaires proviennent du même donneur allogène que les lymphocytes T γδ.

7. Lymphocytes T γδ allogènes dérivés d'un donneur destinés à être utilisés selon l'une quelconque des revendications 1 à 6, comprenant en outre la culture des lymphocytes T γδ avec l'interleukine-2.

8. Lymphocytes T γδ allogènes dérivés d'un donneur destinés à être utilisés selon l'une quelconque des revendications 1 à 7, comprenant en outre la culture des lymphocytes T γδ avec un ou plusieurs inhibiteurs de point de contrôle avant l'administration des lymphocytes T γδ au sujet.

9. Lymphocytes T γδ allogènes dérivés d'un donneur destinés à être utilisés selon la revendication 8, dans lesquels l'un ou plusieurs inhibiteurs de point de contrôle comprend un anticorps anti-PD-1, un anticorps anti-PDL1, un anticorps anti-TIM-3, un anticorps anti-LAG3, un anticorps anti-galectine-9, un anticorps anti-IDO et/ou un anticorps anti-VISTA.

10. Lymphocytes T γδ allogènes dérivés d'un donneur destinés à être utilisés selon l'une quelconque des revendications 1 à 9, comprenant en outre la culture des lymphocytes T γδ avec un ou plusieurs de l'IL-15, d'un anticorps anti-CD277, d'un anticorps anti-TGF-béta, d'un inhibiteur de prostaglandine E₂, de l'adénosine, de l'ostéopontine, de la vitamine C et/ou d'un agent hypométhylant avant l'administration des lymphocytes T γδ au sujet.

11. Lymphocytes T γδ allogènes dérivés d'un donneur destinés à être utilisés selon l'une quelconque des revendications 1 à 10, dans lesquels le sujet et le donneur des lymphocytes T γδ ont un mésappariement d'antigène leucocytaire (HLA) humain complet.

12. Lymphocytes T γδ allogènes dérivés d'un donneur destinés à être utilisés selon l'une quelconque des revendications 1 à 11, comprenant en outre la répétition des étapes (a) et (c) une ou plusieurs fois, les lymphocytes T γδ allogènes dérivés d'un donneur pour chaque administration subséquente étant d'un donneur différent ayant un mésappariement HLA complet par rapport au sujet et au(x) donneur(s) précédent (s).
